# NEUE EUROPÄISCHE PATENTSCHRIFT

(11) **EP 1 855 741 B2**
(45) Veröffentlichungstag und Bekanntmachung des Hinweises auf die Entscheidung über den Einspruch: **05.04.2017**
(45) Hinweis auf die Patenterteilung: 11.02.2009
(21) Anmeldenummer: 06700025.7
(22) Anmeldetag: 11.01.2006
(51) Int. Cl.: A61M 5/315, A61M 5/24

(54) **VERABREICHUNGSVORRICHTUNG MIT ANZEIGETROMMEL**
ADMINISTERING DEVICE COMPRISING A DISPLAY DRUM
DISPOSITIF D'ADMINISTRATION COMPORTANT UN TAMBOUR D'AFFICHAGE

(30) Priorität: 23.02.2005 DE 102005008280
(43) Veröffentlichungstag der Anmeldung: 21.11.2007
(60) Teilanmeldung: 09152374.6
(73) Patentinhaber: TecPharma Licensing AG, 3401 Burgdorf (CH)
(72) Erfinder: KIRCHHOFER, Fritz, CH-3454 Sumiswald (CH)
(74) Vertreter: Schwabe - Sandmair - Marx
(86) Internationale Anmeldenummer: PCT/CH2006/000018
(87) Internationale Veröffentlichungsnummer: WO 2006/089436

(56) Entgegenhaltungen:
- EP-A2- 0 937 476
- WO-A-99/38554
- WO-A1-01/95959
- WO-A1-99/38554
- WO-A1-03/075985
- WO-A1-2004/078239
- WO-A1-2005/046770
- WO-A2-2004/078242
- DD-A1- 234 613
- DE-A1- 10 229 138
- DE-A1- 10 229 138
- DE-A1- 10 232 411
- DE-T2- 69 223 521
- DE-U1- 20 317 377
- US-A- 5 320 609
- US-A- 5 320 609
- US-A- 5 626 566
- US-A- 5 626 566
- US-A1- 2002 052 578
- US-A1- 2005 033 244
- EHRHARDT A. ET AL: 'LUEGER, Lexikon der Technik', DEUTSCHE VERLAG-ANSTALT, STUTTGART Seite 176
- 'BROCKHAUS, Naturwissenschaften und Technik', Bd. 10, 1983, F.A. BROCKHAUS, WIESBADEN Seite 90
- Auszug aus Wikipedia http://en.wikipedia.org/wiki/Complement_(di sambiguation)

## Beschreibung

Die Erfindung betrifft eine Vorrichtung für die Verabreichung eines fluiden Produkts, das insbesondere ein Medikament, ein Diagnosemittel oder ein kosmetisches Produkt sein kann. Bevorzugt wird die Erfindung in der Selbstverabreichung eingesetzt, obgleich sie hierauf nicht beschränkt ist, sondern auch in der Verabreichung durch medizinisch geschultes Personal zum Einsatz gelangen kann.

In Therapien, in denen medizinisch nicht geschulte Personen einen Wirkstoff verabreichen, beispielsweise sich selbst, müssen die verwendeten Verabreichungsvorrichtungen einfach, aber dennoch sicher handhabbar sein. Insbesondere muss sichergestellt sein, dass bei Vorrichtungen, die eine Einstellung der zu verabreichenden Produktdosis erlauben, die eingestellte Dosis zweifelfrei angezeigt wird. In der Diabetestherapie, einem bevorzugten Verwendungsfeld der Erfindung, ist auch zu berücksichtigen, dass die sich das Insulin selbst verabreichenden Personen sehgeschädigt sein können und die eingestellte Dosis daher besonders deutlich und eindeutig auf einer Dosisskala ablesbar sein sollte. Der guten Ablesbarkeit zuträglich ist es, wenn die Dosisskala auf einer Anzeigetrommel aufgetragen ist, die rotatorisch und translatorisch bewegbar ist, da die Dosisskala in diesem Fall längs und in Drehrichtung der Anzeigetrommel erstreckt werden kann.

Vorrichtungen der genannten Art sind beispielsweise aus der WO 99/38554 A1 bekannt. Die Vorrichtungen sind komplex und benötigen Verdrehsicherungen, die eine Drehbewegung einer Kolbenstange in die eine Drehrichtung zulassen, aber in die andere verhindern und zusätzlich auf zu übertragende oder gerade noch übertragbare Drehmomente abgestimmt sein müssen.

Die WO 2004/078239 A1 offenbart eine Verabreichungsvorrichtung mit Anzeigetrommel bestehend aus einem ersten Förderglied, das mit einem zweiten Förderglied in einem ersten Gewindeeingriff steht, und einer Anzeigetrommel, die mit einem inneren Gehäuseteil in einem zweiten Gewindeeingriff steht, wobei beide Gewindeeingriffe die gleiche Gewindesteigung aufweisen.

Aus der WO 03/075985 A1 ist eine einfachere Vorrichtung der genannten Art bekannt. Wie bei den Vorrichtungen der WO 99/38554 bedarf es allerdings zweier Spindeltriebe, die beide nicht selbsthemmend gebildet sind, so dass bei Einstellung einer nur kleinen Dosis auch nur ein kurzer Hub der Anzeigetrommel zur Verfügung steht.

Aus der US 2002/0052578 A1 ist eine Verabreichungsvorrichtung mit den Merkmalen a) bis f) des Anspruchs 1 bekannt. Das Betätigungselement dieser Verabreichungsvorrichtung ist mit dem zweiten Kupplungsglied verbunden, so dass das zweite Kupplungsglied und das Betätigungselement in und gegen die Förderrichtung relativ zueinander nicht beweglich sind. Das Betätigungselement ist relativ zum ersten Kupplungsglied der Verabreichungsvorrichtung um die Förderachse drehbar.

Es ist eine Aufgabe der Erfindung, bei einer Verabreichungsvorrichtung der genannten Art die bei Betätigung auftretende Reibung zu vermindern.

Die Aufgabe wird durch den Gegenstand des Anspruchs 1 gelöst.

Die Erfindung betrifft eine Vorrichtung zur Verabreichung eines fluiden Produkts, die ein Gehäuse mit einer Aufnahme für das Produkt, eine Fördereinrichtung zur Förderung des Produkts und eine Dosiereinrichtung für die Einstellung einer zu verabreichenden Produktdosis und Anzeigen der eingestellten Produktdosis umfasst. Das Gehäuse bildet eine Aufnahme für das Produkt, vorzugsweise eine Aufnahme für ein mit dem Produkt gefülltes Behältnis. Grundsätzlich könnte die Aufnahme jedoch auch selbst unmittelbar das Produktbehältnis bilden. Die Fördereinrichtung umfasst ein erstes Förderglied, das relativ zu dem Gehäuse in eine Förderrichtung bewegbar ist, um die eingestellte Produktdosis in einem entsprechend der Produktdosis voreingestellten Förderhub auszuschütten. Bei dem Förderhub handelt es sich vorzugsweise um eine translatorische Bewegung des ersten Förderglieds in die Förderrichtung, vorzugsweise um eine Linearbewegung entlang einer Förderachse. Das erste Förderglied kann insbesondere als Kolbenstange gebildet sein, die in solch einer Ausbildung mit einem Kolben der Fördereinrichtung verbunden ist, beispielsweise mit dem Kolben in einem Stück gebildet oder mit dem Kolben verschraubt oder verschnappt ist, oder bei dem Förderhub lose gegen eine Rückseite des Kolbens drückt. Die Fördereinrichtung umfasst ferner ein zweites Förderglied, das mit dem ersten Förderglied in einem ersten Gewindeeingriff ist, in dem es für die Einstellung der Produktdosis relativ zu dem ersten Förderglied und dem Gehäuse drehbar und für die Ausschüttung der eingestellten Produktdosis relativ zu dem Gehäuse in die Förderrichtung translatorisch bewegbar ist. Falls die Fördereinrichtung für die Förderung des Produkts einen axial bewegbaren Kolben aufweist, wie dies bevorzugt wird, kann das erste Förderglied insbesondere eine Kolbenstange bilden. In derartigen Ausführungen kann das erste Förderglied grundsätzlich aber auch nur ein Übertragungsglied sein, das eine Förderbewegung des zweiten Förderglieds erst auf eine Kolbenstange überträgt.

Die Anzeigetrommel ist in einem zweiten Gewindeeingriff relativ zu dem Gehäuse drehbar sowie in und gegen die Förderrichtung translatorisch bewegbar. Vorzugsweise ist die Anzeigetrommel mit dem Gehäuse oder einer mit dem Gehäuse verbundenen Zwischenstruktur in dem zweiten Gewindeeingriff. Falls der zweite Gewindeeingriff mit einer Zwischenstruktur gebildet ist, ist die Zwischenstruktur mit dem Gehäuse vorzugsweise so verbunden, dass sie sich entweder bei der Einstellung der Dosis oder bei der Förderbewegung oder vorzugsweise in beiden Fällen relativ zu dem Gehäuse nicht bewegt.

Nach der Erfindung umfasst die Vorrichtung ferner eine Kupplung, welche die Anzeigetrommel mit dem zweiten Förderglied verdrehgesichert koppelt, wobei der für die verdrehsichere Kopplung von der Kupplung gebildete Kupplungseingriff sich automatisch löst, wenn die Vorrichtung für die Verabreichung betätigt wird. Vorteilhafterweise wird der Kupplungseingriff gelöst bevor der Förderhub des zweiten Förderglieds einsetzt. Indem das zweite Förderglied relativ zu dem Gehäuse in und gegen die Förderrichtung bewegbar ist, mit dem ersten Förderglied einen ersten Spindeltrieb bildet und während der Einstellung der Produktdosis über einen lösbaren Kupplungseingriff mit der Anzeigetrommel gekoppelt ist, können die Gewindesteigungen der beiden Spindeltriebe, nämlich der mit dem zweiten Förderglied gebildete erste Spindeltrieb und der mit der Anzeigetrommel gebildete zweite Spindeltrieb, zueinander auf einfache Weise in weiten Grenzen variiert werden, wobei die Gewindesteigung des zweiten Gewindeeingriffs größer ist als die Gewindesteigung des ersten Gewindeeingriffs. So kann der mit der Anzeigetrommel gebildete Spindeltrieb insbesondere eine große Gewindesteigung und der mit dem zweiten Förderglied gebildete Spindeltrieb eine besonders kleine Gewindesteigung aufweisen. Es wird somit ein kleiner Förderhub mit einem großen Anzeigehub kombiniert werden. So kann die Gewindesteigung im zweiten Gewindeeingriff beispielsweise drei-, vier- oder fünfmal so groß sein wie die Gewindesteigung im ersten Gewindeeingriff.

Der Gewindeeingriff der Förderglieder ist vorzugsweise selbsthemmend, was vorteilhaftenveise dadurch erreicht wird, dass die Steigung der im Gewindeeingriff befindlichen Gewinde ausreichend klein ist. Stattdessen oder zusätzlich kann das zweite Förderglied durch einen lösbaren Rasteingriff an einer unerwünschten Drehbewegung während des Förderhubs gehindert werden. Der Winkelabstand der Drehrastpositionen solch eines Rasteingriffs entspricht der Vergrößerung oder Verkleinerung der Dosis um eine einstellbare Einheit.

Die Kupplung wird mittels einer Feder in den Kupplungseingriff gespannt und umfasst hierfür ein erstes Kupplungsglied und ein zweites Kupplungsglied sowie die Feder, welche die Kupplungsglieder miteinander in den Kupplungseingriff spannt. Zumindest in Ausgestaltungen, in denen die für die Ausführung des Förderhubs erforderliche Kraft manuell aufgebracht wird, wird es bevorzugt, wenn der Kupplungseingriff durch die für den Förderhub aufzubringende Kraft gelöst wird. Vorteilhafterweise wird die den Förderhub bewirkende Betätigungskraft in die Förderrichtung ausgeübt, und die Feder spannt eines der Kupplungsglieder in oder vorzugsweise gegen die Förderrichtung in den Kupplungseingriff mit dem anderen. Der Kupplungseingriff, d.h. die durch die Kupplungsglieder hergestellte Verdrehsicherung, kann rein auf einem Reibschluss beruhen, vorzugsweise beruht sie jedoch auf Formschluss oder umfasst zumindest eine Formschlussverbindung der Kupplungsglieder. Der Kupplungseingriff wird vorzugsweise durch eine in die Förderrichtung erfolgende Bewegung, die eines der Kupplungsglieder relativ zu dem anderen ausführt, gelöst. Vorteilhafterweise ist eines der Kupplungsglieder relativ zu dem anderen in die Förderrichtung längs der Förderachse aus dem Kupplungseingriff bewegbar.

Für die Betätigung umfasst die Vorrichtung ein Betätigungselement, durch dessen Betätigung der Kupplungseingriff gelöst wird. Wenigstens eines aus Anzeigetrommel und zweitem Förderglied, vorzugsweise die Anzeigetrommel und noch bevorzugter auch das zweite Förderglied, ist relativ zu dem Betätigungselement drehbar. Das Betätigungselement ist vorzugsweise permanent mit einem der beiden Kupplungsglieder verbunden. Vorzugsweise ist es in und gegen eine Richtung, in die das betreffende Kupplungsglied aus dem Kupplungseingriff bewegt wird, nicht beweglich mit dem betreffenden Kupplungsglied verbunden. Bei der Betätigung gelangt das Betätigungselement vorzugsweise gegen das andere der Kupplungsglieder in einen Kontakt. Das Betätigungselement und das andere der Kupplungsglieder bilden für den Kontakt vorzugsweise reibarme Kontaktflächen um einer im Betätigungskontakt möglicherweise auftretenden Relativdrehung nur geringe Reibungskräfte entgegenzusetzen.

Die Feder der Kupplung ist vorzugsweise eine mechanische Feder. Sie wirkt vorteilhafterweise als Druckfeder. Alternativ kann sie aber beispielsweise auch als Zugfeder wirken, welche die Kupplungsglieder mit ihrer Federkraft in den Kupplungseingriff zieht. In der bevorzugten Ausbildung als Druckfeder wirkt sie zum einen gegen eines der Kupplungsglieder und zum anderen vorzugsweise gegen das zweite Förderglied. Als Druckfeder kann sie alternativ auch als Druckgasfeder, beispielsweise Druckluftfeder, gebildet sein.

In bevorzugten Ausführungen ist eine Ausgleichseinrichtung vorgesehen, die in Förderrichtung längenvariabel ist und das zweite Förderglied mit der Anzeigetrommel verdrehgesichert verbindet, wenn der Kupplungseingriff besteht. Die Ausgleichseinrichtung umfasst bevorzugterweise eine Feder, die so angeordnet ist, dass die Länge der Ausgleichseinrichtung nur gegen eine rückstellende Elastizitätskraft der Feder verringerbar ist. Die Feder ist vorzugsweise als mechanische Feder gebildet, könnte grundsätzlich aber auch eine pneumatische Feder sein. Das zu der Feder der Kupplung gesagte gilt für die Feder der Ausgleichseinrichtung ebenfalls. Die Feder kann insbesondere die im Zusammenhang mit der Kupplung beschriebene Feder sein. Obgleich weniger bevorzugt, kann die dem Längenausgleich dienende Feder aber auch zusätzlich zu der Feder der Kupplung vorgesehen sein.

Die Ausgleichseinrichtung umfasst bevorzugterweise wenigstens zwei in Förderrichtung einander überlappende und relativ zueinander verdrehgesichert geführte Ausgleichsstrukturen. Vorzugsweise sind die wenigstens zwei Ausgleichsstrukturen unmittelbar aneinander verdrehgeführt. Bei bestehendem Kupplungseingriff ist eine der Ausgleichsstrukturen mit der Anzeigetrommel und die andere mit dem zweiten Förderglied verdrehgesichert verbunden. Die Ausgleichseinrichtung ist in bevorzugten Ausführungen als Teleskop gebildet. Die Ausgleichsstmkturen bilden in derartigen Ausführungen je einen in Förderrichtung erstreckten Teleskopabschnitt. Die Feder der Ausgleichseinrichtung stützt sich in vorteilhaften Ausbildungen in die Förderrichtung an der einen und gegen die Förderrichtung an der anderen der Ausgleichsstrukturen ab, so dass die Federkraft trachtet, die Ausgleichsstrukturen auseinander zu treiben. Bevorzugt weist die Ausgleichseimichtung nur genau zwei in Förderrichtung relativ zueinander bewegbare Ausgleichsstrukturen auf. Die Ausgleichsstnikturen sind in sich in Förderrichtung vorteilhafterweise steif, könnten aber auch selbst in Förderrichtung eine Elastizität aufweisen, so dass sogar auf eine separate Feder verzichtet werden könnte. So könnte die Ausgleichseinrichtung durchaus als Federbalg gebildet sein, der gleichzeitig auch eines der Kupplungsglieder bilden kann.

Die Ausgleichseinrichtung ist besonders vorteilhaft in Kombination mit der Kupplung. Sie ist für eine Aufrechterhaltung der verdrehgesicherten Verbindung zwischen der Anzeigetrommel und dem zweiten Förderglied aber auch als solche bereits vorteilhaft, d.h. für eine Vorrichtung, die zwar die Merkmale a) bis d), nicht jedoch das Merkmal e) von Anspruch 1 aufweist.

In bevorzugter Ausführung umfasst die Vorrichtung eine Führung für eine verdrehgesicherte Linearführung des ersten Förderglieds in Förderrichtung. Auf ein im Förderstrang nachgeordnetes Förderglied, beispielsweise einen Kolben, kann von solch einem ersten Förderglied vorteilhafterweise kein Drehmoment übertragen werden.

Bevorzugterweise ist eine Rückhalteeinrichtung vorgesehen, die in einem Eingriff mit dem ersten Förderglied eine Bewegung des ersten Förderglieds gegen die Förderrichtung verhindert. Die Rückhalteeinrichtung und das erste Förderglied, soweit sein Zusammenwirken mit der Rückhalteeinrichtung betroffen ist, können insbesondere wie von Zahnstangenpens her bekannt gebildet sein. So kann das erste Förderglied beispielsweise als Zahnstange mit wenigstens einer Sägezahnreihe gebildet sein, in welche die Rückhalteeinrichtung mit einer federnd biegbaren Rückhaltezunge eingreifend eine Bewegung des ersten Förderglieds gegen die Förderrichtung verhindert, die Bewegung in die Fördewichtung jedoch zulässt.

Die Vorrichtung kann insbesondere ein Injektionsgerät sein, vorteilhafterweise für subkutane Verabreichungen mittels Injektionsnadel oder auch für nadellose Druckinjektoren. Das Injektionsgerät ist bevorzugt ein Injektionspen. Wie bereits in Verbindung mit der Rückhalteeinrichtung erwähnt, kann die Vorrichtung in der Art eines Zahnstangenpens gebildet sein, wobei das erste Förderglied allerdings als kombinierte Zahn-Gewinde-Stange gebildet ist oder zumindest einen entsprechenden Abschnitt aufweist. Aufgrund ihres einfachen Aufbaus und daher günstigen Preises bietet sich die Vorrichtung auch als Einweggerät an, das nach Entleerung des Produktbehältnisses entsorgt wird.

Bevorzugte Merkmale der Erfindung werden auch in den Unteransprüchen und deren Kombinationen beschrieben.

Ein Ausführungsbeispiel der Erfindung wird nachfolgend anhand von Figuren erläutert. An dem Ausführungsbeispiel offenbar werdende Merkmale bilden je einzeln und in jeder Merkmalskombination die Gegenstände der Ansprüche und auch die vorstehend beschriebenen Ausgestaltungen vorteilhaft weiter. Es zeigen:
- Figur 1: eine erfindungsgemäße Vorrichtung in einem Zustand der Nulldosis-Einstellung in einer Ansicht auf eine Dosisanzeige,
- Figur 2: die Vorrichtung in einem Zustand der Maximaldosis-Einstellung in der Ansicht auf die Dosisanzeige,
- Figur 3: die Vorrichtung im Zustand der Maximaldosis-Einstellung in einem. Längsschnitt,
- Figur 4: die Vorrichtung im Zustand der Nulldosis-Einstellung im gleichen Längsschnitt und
- Figur 5: einen proximalen Abschnitt der Vorrichtung in einem Zustand der Maximaldosis-Einstellung in dem Längsschnitt der Figuren 3 und 4.

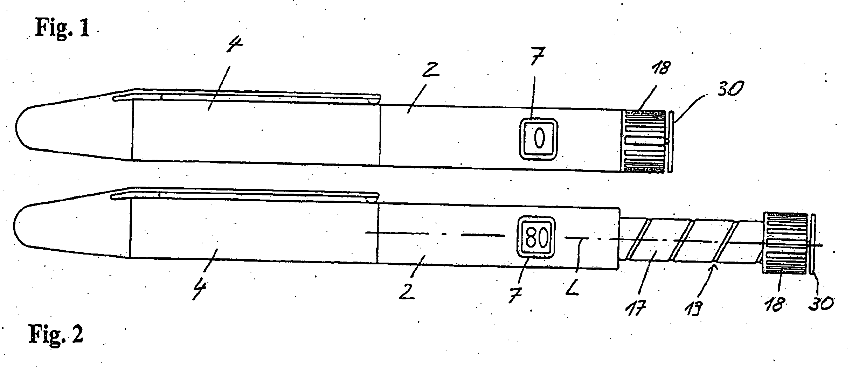

Figur 1 zeigt eine Vorrichtung für die dosierte Verabreichung eines fluiden Produkts, beispielsweise Insulin, ein Osteoporosepräparat oder ein Wachstumshormon. Die Vorrichtung ist als schlanker Injektionspen gebildet. Sie umfasst ein Gehäuse, von dem ein proximaler Gehäuseabschnitt 2 erkennbar ist.. Eine Kappe 4 deckt einen distalen Gehäuseabschnitt ab. Die Vorrichtung befindet sich in einem Ausgangszustand, den sie vor Einstellung einer zu verabreichenden Produktdosis oder nach der Verabreichung einnimmt. Zu erkennen ist ein Dosierglied 18 für die Einstellung der Produktdosis und eine Dosisanzeige mit einem Fenster 7. Das Fenster 7 ist als Durchbrechung des Gehäuseabschnitts 2 gebildet und wird von einer durchsichtigen Abdeckung, beispielsweise eine Lupe abgedeckt; grundsätzlich kann das Fenster 7 auch einfach nur eine Durchbrechung sein, durch dies auf einer Dosisskala die mittels des Dosierglieds 18 eingestellte Dosis ablesbar ist. Im Ausgangszustand der Figur 1 zeigt die Dosisanzeige die Dosis "Null" an. Ein tellerförmiges Betätigungselement 30 bildet ein proximales Ende der Vorrichtung.

Figur 2 zeigt die Vorrichtung in der gleichen Ansicht wie Figur 1, allerdings nach Einstellung einer mit der Vorrichtung pro Injektion maximal verabreichbaren Produktdosis. Wie an der Dosisanzeige ablesbar, entspricht die maximale Produktdosis beispielhaft 80 Dosiseinheiten des Produkts. Das Dosierglied 18 ist als Drehknopf gebildet. Durch eine Dosierdrehbewegung des Dosierglieds 18 wird die Dosis eingestellt. Durch die Dosierdrehbewegung wird eine Anzeigetrommel 17 längs einer zentralen Längsachse L der Vorrichtung aus dem Gehäuseabschnitt 2 herausgedreht. Die Dosisskala ist auf der äußeren Umfangsfläche der Anzeigetrommel 17 spiralig umlaufend aufgetragen. Die Anzeigetrommel 17 ist um die zentrale Längsachse L spiralig umlaufend mit einem Gewinde 19 versehen, das mit einem entsprechenden Innengewinde des Gehäuseabschnitts 2 in einem Gewindeeingriff steht, so dass die Anzeigetrommel 17 mit dem Gehäuseabschnitt 2 einen Spindeltrieb bildet. Die Steigung des Gewindeeingriffs bezüglich der zentralen Längsachse L ist so groß, dass eine Selbsthemmung nicht auftreten kann, wenn die Anzeigetrommel 17 durch Druck gegen das Betätigungselement 30 in den Gehäuseabschnitt 2 gedrückt wird.

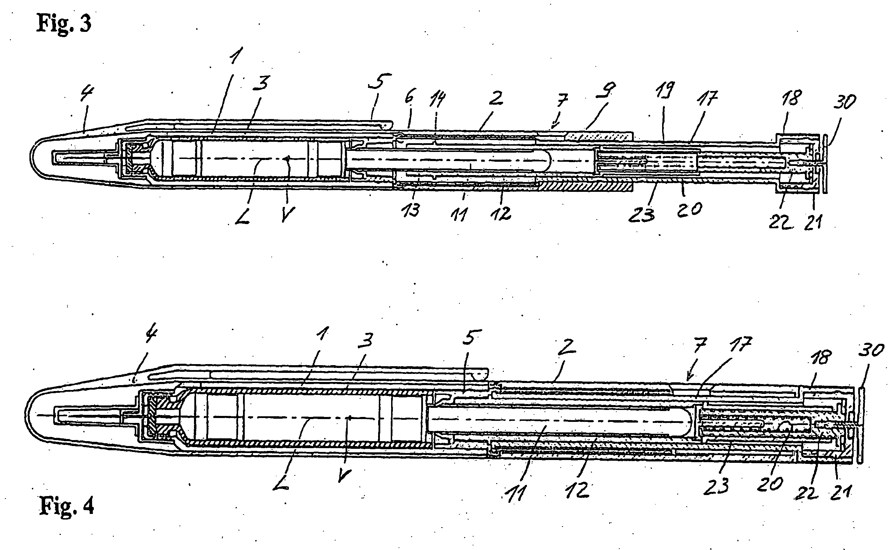

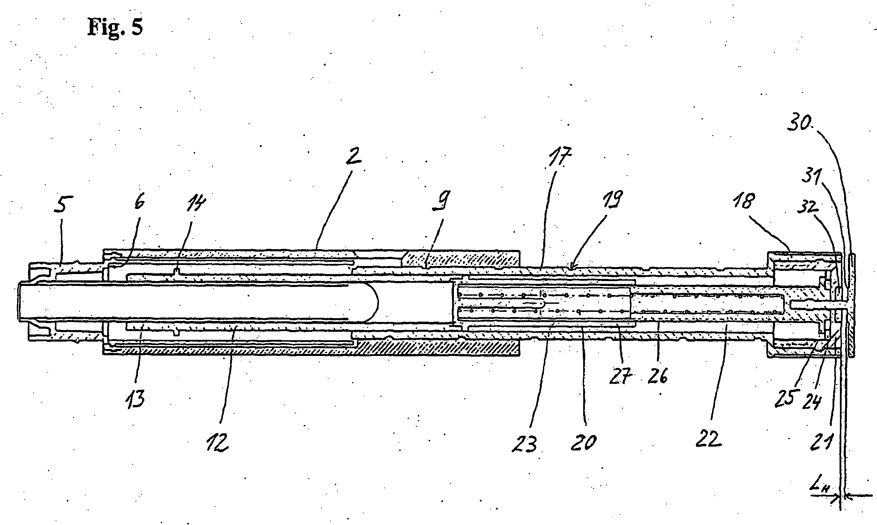

Figur 3 zeigt die Vorrichtung im Zustand der Figur 2, d.h. im Zustand der Maximaldosis-Einstellung, in einem Längsschnitt, der die zentrale Längsachse L der Vorrichtung enthält. Figur 4 zeigt die Vorrichtung im Zustand der Figur 1, d.h. in der Nulldosis-Einstellung, im gleichen Längsschnitt. Figur 5 zeigt wiederum im gleichen Längsschnitt, jedoch vergrößert, den proximalen Teil der Vorrichtung im Zustand der Maximaldosis-Einstellung.

Der distale Gehäuseabschnitt 1 und der damit unbeweglich verbundene proximale Gehäuseabschnitt 2 bilden ein Gehäuse 1, 2 der Vorrichtung. Der distale Gehäuseabschnitt 1 bildet eine Aufnahme für ein mit dem Produkt gefülltes Behältnis 3, das von einer Ampulle gebildet wird, wie sie beispielsweise aus der Diabetestherapie für Injektionspens bekannt ist. Ein distaler Auslass der Ampulle wird von einem Septum verschlossen. Eine längs der Längsachse L angeordnete Injektionskanüle durchragt das Septum. In dem Behältnis 3 ist ein Kolben längs der Längsrichtung L in eine Förderrichtung V auf den Auslass zu bewegbar aufgenommen. Die Längsachse L bildet auch eine Förderachse der Vorrichtung und wird im folgenden auch so bezeichnet.

Eine Kolbenstange treibt den Kolben längs der Förderachse L in die Förderrichtung V. Die Kolbenstange bildet ein erstes Förderglied 11 einer Fördereinrichtung, die auch den Kolben und ferner ein das erste Förderglied 11 antreibendes zweites Förderglied 12 umfasst. Das erste Förderglied 11 ist in den Figuren 3, 4 und 5 nicht eingezeichnet, das Bezugszeichen "11" deutet auf den Einbauort. Das erste Förderglied 11 ist mit dem zweiten Förderglied 12 in einem Gewindeeingriff, wobei die Förderachse L die Gewindeachse ist. Das erste Förderglied 11 ist des weiteren mit einer Rückhalteeinrichtung 5 in einem Eingriff, die in und gegen die Förderrichtung V nicht beweglich mit dem Gehäuseabschnitt 2 verbunden ist. Die Rückhalteeinrichtung 5 bildet zwei Rückhaltezungen, die in Längsnuten des ersten Förderglieds 11 eingreifen und eine Bewegung des ersten Förderglieds 11 gegen der Förderrichtung V blockieren. Das erstes Förderglied 11 ist für den Rückhalteeingriff in den beiden Nuten je mit einer Sägezahnreihe versehen. Das Zusammenwirken des ersten Förderglieds 11 und der Rückhalteeinrichtung 5 entspricht dem bekannter Zahnstangenpens. Ein Beispiel eines derartigen Pens wird in der DE 102 32 411 A1 beschrieben, auf die diesbezüglich verwiesen sei.

Das erste Förderglied 11 ist des weiteren relativ zu dem Gehäuseabschnitt 2 um die Förderachse L nicht drehbar, wobei eine hierfür gebildete Verdrehsicherung eine Drehbewegung in beide Drehrichtungen in allen Betriebszuständen der Vorrichtung blockiert. Die Rückhalteeinrichtung 5 kann gleichzeitig auch die Verdrehsicherung bilden, eine Verdrehsicherung kann jedoch auch zusätzlich vorgesehen sein.

Das zweite Förderglied 12 ist hülsenfönnig, umgibt das erste Förderglied 11 und weist an seinem distalen Ende ein Innengewinde 13 auf, das mit einem Außengewinde des ersten Förderglieds 11 in dem Gewindeeingriff ist. Das Innengewinde 13 und das Außengewinde des ersten Förderglieds 11 sind Feingewinde mit einer entsprechend geringen Gewindesteigung. Das zweite Förderglied 12 bildet ebenfalls noch im distalen Endbereich einen nach radial außen abragenden Förderanschlag 14, der im Zusammenwirken mit einem Gegenanschlag 6, den die Rückhalteeim-ichtung 5 bildet, die Förderbewegung des zweiten Förderglieds 12 und damit wegen des Gewindeeingriffs auch des ersten Förderglieds 11 in die Förderrichtung V begrenzt. Den Anschlag 14 kann ein umlaufender Flansch oder eine Mehrzahl einzelner Nocken oder gegebenenfalls auch nur ein einzelner Nocken bilden, falls der Gegenanschlag 6 um die Förderachse L umläuft.

Über den Gewindeeingriff der Förderglieder 11 und 12 hinaus ist als weiterer, zweiter Gewindeeingriff derjenige zwischen der Anzeigetrommel 17 und dem Gehäuseabschnitt 2 gebildet, nämlich zwischen dem Außengewinde 19 der Anzeigetrommel 17 und einem Innengewinde 9 des Gehäuseabschnitts 2. Die Förderachse L ist auch die Gewindeachse des zweiten Gewindeeingriffs. Zwischen dem zweiten Förderglied 12 und dem Gehäuseabschnitt 2 verbleibt ein Ringspalt, in den die Anzeigetrommel 17 im zweiten Gewindeeingriff bei der Ausschüttung des Produkts einfährt und aus dem sie bei der Einstellung der Dosis ausfährt. Beim Ein- und Ausfahren gleitet sie über die von dem Anschlag 14 abgesehen glatte Mantelaußenfläche des zweiten Förderglieds 12. Die Anzeigetrommel 17 ist somit radial beidseitig geführt, zum einen im zweiten Gewindeeingriff 9, 19 und zum anderen durch das zweite Förderglied 12. Indem die Anzeigetrommel 17 das zweite Förderglied 12 eng anliegend umgibt, kann der Pen sehr schlank gehalten werden.

Das zweite Förderglied 12 ist für die Einstellung der Dosis über eine Kupplung verdrehgesichert mit dem Dosierglied 18 verbunden. Das Dosierglied 18 und die Anzeigetrommel 17 wiederum sind in einem Stück als Hülsenkörper gebildet mit der Anzeigetrommel 17 als langgestreckter distaler Hülsenabsclnitt und dem Dosierglied 18 als demgegenüber breiterer, aber axial kürzerer proximaler Endabschnitt.

Die Kupplung umfasst ein erstes Kupplungsglied 21, ein zweites Kupplungsglied 22 und eine Feder 20, die als Spiralfeder gebildet ist und als Druckfeder wirkend das zweite Kupplungsglied 22 in einen Kupplungseingriff mit dem ersten Kupplungsglied 21 drückt. Im Kupplungseingriff sind die Kupplungsglieder 21 und 22 formschlüssig bezüglich der Förderachse L verdrehgesichert miteinander verbunden. Das erste Kupplungsglied 21 ist in den das Dosierglied 18 bildenden Hülsenabsclnitt des Hiilsenköniers 17, 18 eingesetzt und mit dem Hülsenkörper 17, 18 sowohl verdrehgesichert als auch axial unbeweglich verbunden. Das erste Kupplungsglied 21 ist topfformig mit einem Boden am proximalen Ende und einer von dem Boden umlaufend in die distale Richtung aufragenden Wand geformt. Es schließt somit den Hülsenkörper 17, 18 an dessen proximalen Ende ab. Für den Kupplungseingriff sind das erste Kupplungsglied 21 mit Kupplungsrippen, -nocken, -nuten 24 oder dergleichen und das zweite Kupplungsglied 22 mit entsprechenden Gegennuten, -rippen oder-nocken 25 oder dergleichen ausgestattet, die ineinandergreifend eine Drehbewegung eines der Kupplungsglieder 21 und 22 relativ zu dem anderen verhindern. Grundsätzlich genügt für den Kupplungseingriff natürlich auch nur ein einziges Paar aus beispielsweise Kupplungsrippe 24 und Kupplungsnut 25 als Eingriffselemente.

Das zweite Kupplungsglied 22 ist in Doppelfunktion auch Bestandteil einer Ausgleichseinrichtung, welche das Kupplungsglied 22 als eine erste Ausgleichsstruktur und eine damit zusammenwirkende zweite Ausgleichsstruktur 23 umfasst. Auch die Feder 20 erfüllt eine Doppelfunktion, zum einen als Kupplungsfeder der Kupplung und zum anderen als Ausgleichsfeder der Ausgleichseinrichtung. Die beiden Ausgleichsstrukturen 22 und 23 bilden ein Teleskop. Sie sind in und gegen die Förderrichtung V längs der Förderachse L aneinander verdrehgesichert linear geführt, wobei die Ausgleichsstruktur 22 einen inneren und die Ausgleichsstruktur 23 einen äußeren Teleskopabschnitt bildet. Für die Verdrehsicherung ist die Ausgleichsstruktur 22 an ihrer Mantelaußenfläche mit Fülu-ungselementen 26 und die Ausgleichsstruktur 23 an ihrer Mantelinneniläche mit Fühnmgsgegenelementen 27 versehen. Die Führungselemente 26 und 27 sind in der Art einer Nut- und Federführung gebildet. Im Grunde könnte die Führung auch mittels nur einem einzigen Führungselement und einem einzigen Führungsgegenelement gebildet sein. Wie die Figuren 3 bis 5 erkennen lassen, überlappen die beiden Ausgleichstrukturen 22 und 23 einander im Zustand der Nulldosis-Einstellung nahezu vollständig, während die axiale Überlappung im Zustand der Maximaldosis-Einstellung minimal ist und gerade noch die verdrehgesicherte Verbindung zwischen der Anzeigetrommel 17 und dem Dosierglied 18 einerseits und dem zweiten Förderglied 12 andererseits gewährleistet. Die Ausgleichsstruktur 23 ist topfförniig mit einem Boden am distalen Ende und einer von dem Boden umlaufend in die proximale Richtung aufrageliden Hülsenwand. Die Feder 20 stützt sich in die Förderrichtung V an dem Boden der Ausgleichsstruktur 23 ab. Von dem Boden ragt ferner zentral eine Axialführung für die Feder 20 auf. Die Feder 20 drückt die Ausgleichsstruktur 23 in Förderrichtung V auf Anschlag gegen das zweite Förderglied 12. Ferner sind das zweite Förderglied 12 und die Ausgleichsstruktur 23 miteinander in einem verdrehgesicherten Eingriff bezüglich der Förderachse L. Für den verdrehgesicherten Eingriff weist das zweite Förderglied 12 an seinem proximalen Ende an einer Mantelinnenfläche axial kurze Eingriffselemente auf, die mit an der Mantelaußenfläche der Ausgleichsstruktur 23 gebildeten Gegenelementen in dem verdrehgesicherten Eingriff sind. Die Ausgleichsstruktur 23 ist im Ausführungsbeispiel ein separates Bauteil, sie kann alternativ jedoch in einem Stück mit dem zweiten Förderglied 12 geformt sein. Die Formung als separates Bauteil ist fertigungstechnisch jedoch vorteilhaft.

Das Betätigungselement 30 bildet einen tellerförmigen Abschluss der Vorrichtung. Von der tellerförmigen Struktur ragt in Förderrichtung V ein stiftförmiger Verbindungsabschnitt durch den Boden des ersten Kupplungsglieds 21 in das Innere des Dosierglieds 18 hinein und ist dort in einem Bodenabschnitt des ebenfalls topfförmigen zweiten Kupplungsglieds 22 verankert, so dass das Kupplungsglied 22 und das Betätigungselement 30 in und gegen die Förderrichtung V relativ zueinander nicht beweglich sind. Vorzugsweise sind sie relativ zueinander um die Förderachse L drehbar; grundsätzlich können sie jedoch auch verdrehgesichert miteinander verbunden sein. Das Betätigungselement 30 ist gemeinsam mit dem zweiten Kupplungsglied 22 gegen die rückstellende Elastizitätskraft der Feder 20 relativ zu dem ersten Kupplungsglied 21 in die Förderrichtung V bewegbar. Ferner ist es zumindest relativ zu dem ersten Kupplungsglied 21 um die Förderachse L drehbar.

Wie am besten in Figur 5 zu erkennen ist, verbleibt von dem Verbindungsabschnitt des Betätigungselements 30 abgesehen zwischen dem Betätigungselement 30 und dem ersten Kupplungsglied 21 axial ein lichter Abstand L_{H}. Der Abstand L_{H} besteht zwischen einander axial gegenüberliegenden Kontaktflächen des Betätigungselements 30 und des Kupplungsglieds 21. Die Kontaktfläche des Betätigungselements 30 ist mit 31 bezeichnet und ist um den Verbindungsabschnitt des Betätigungselements 30 zentral als in die distale Richtung aufragende, erhabene Fläche gebildet. Zur Bildung der Kontaktgegenfläche ist in das Kupplungsglied 21 komplementär zur Kontaktfläche 31 ein Kontaktelement 32 eingesetzt. Die Kontaktfläche 31 und die Gegenfläche des Kontaktelements 32 sind besonders reibarm gebildet, beispielsweise aus Teflon.

Nachfolgend wird die Funktion der Vorrichtung bei der Einstellung der Dosis und deren Verabreichung anhand der Figuren 3 und 4 beschrieben, wobei stets auch auf Figur 5 verwiesen sei:

Die Vorrichtung gelangt im Zustand der Nulldosis-Einstellung, wie Figur 4 ihn zeigt, zum Benutzer. In der Aufnahme des distalen Gehäuseabschnitts 1 ist das volle Behältnis 3 eingesetzt. Für eine erste Injektion zieht der Benutzer die Kappe 4 ab und entfernt einen die Injektionsnadel umgebenden Nadelschutz.

Vor Einstellung der gewünschten Dosis kann aus Sicherheitsgründen wieder die Kappe 4 aufgesteckt werden. Zum Einstellen der Dosis hält der Benutzer die Vorrichtung im Bereich des Gehäuses 1, 2 oder bei aufgesetzter Kappe 4 jedenfalls im Bereich des Gehäuseabschnitts 2 und verdreht das Dosierglied 18, bis die gewünschte Dosis eingestellt ist. Aufgrund der Dosierdrehbewegung fährt die Trommel 17 im zweiten Gewindeeingriff 9, 19 in die proximale Richtung aus dem Gehäuseabschnitt 2 heraus. Im Fenster 7 ist die der momentanen Dosierposition entsprechende Dosis ablesbar. Die Dosilerdrehbewegung erfolgt in diskreten Schritten zwischen Rastpositionen. Dabei ist bei jedem Rastvorgang ein deutliches Klickgeräusch wahmelunbar. Die Maximaldosis-Einstellung wird durch einen Anschlag der Anzeigetrommel 17 und einen Gegenanschlag des Gehäuseabschnitts 2 bestimmt. Zwischen den beiden Extremeinstellungen der Nulldosis und der Maximaldosis kam das Dosierglied 18 und damit gemeinsam die Anzeigetrommel 17 in beide Drehrichtungen beliebig von einer Rastposition in die nächste verdreht werden, so dass Dosiskon-ekturen ohne weiteres möglich sind.

Bei dem Ausfahren von Anzeigetrommel 17 und Dosierglied 18 wird das zweite Kupplungsglied 22 von der Feder 20 permanent im Kupplungseingriff mit dem ersten Kupplungsglied 21 gehalten. Ferner ist das Kupplungsglied 22 während der Dosierdrehbewegung permanent im verdrehgesicherten Eingriff mit der Ausgleichsstruktur 23, die ihrerseits von der Feder 20 im verdrehgesicherten Eingriff mit dem zweiten Förderglied 12 gehalten wird. Der rotatorische Anteil der Dosierdrehbewegung wird somit auf das zweite Förderglied 12 übertragen. Da das erste Förderglied 11 relativ zu dem Gehäuse 1, 2 nicht drehbar ist und ferner von der Rückhalteeinrichtung 5 an einer Bewegung gegen die Förderrichtung V gehindert wird, schraubt sich das zweite Förderglied 12 im Gewindeeingriff mit dem ersten Förderglied 11 relativ zu diesem gegen die Förderrichtung V. Bei diesem Dosierhub, den das zweite Förderglied 12 relativ zu dem ersten Förderglied 11 ausführt, verändert sich der axiale Abstand zwischen dem Anschlag 14 und dem Gegenanschlag 6. Dieser axiale Abstand entspricht der Länge des Dosierhubs und entsprechend auch der Länge des Förderhubs.

Da die Gewindesteigung im zweiten Gewindeeingriff 9, 19 größer ist als im Gewindeeingriff der Förderglieder 11 und 12, im ausgeführten Gerät etwas mehr als viermal so groß, absolviert die Anzeigetrommel 17 bei der Dosiseinstellung bei gleichem Drehwinkel einen entsprechend dem Verhältnis der Steigungen längeren Hub. Entsprechend groß und ausreichend voneinander beabstandet sind die Dosiswerte auf der Dosisskala.

Der axiale Dosierhub der Anzeigetrommel 17 und des Dosierglieds 18, d.h. der translatorische Anteil der Dosierbewegung, ist länger als der Dosierhub des zweiten Förderglieds 12. Die Ausgleichseinrichtung 20, 22 und 23 gleicht die Längendifferenz aus, so dass im Kupplungseingriff zwischen den durch Anschläge begrenzten Extremeinstellungen, der Nulldosis-Einstellung der Maximaldosis-Einstellung, stets die verdrehgesicherte Verbindung zwischen der Anzeigetrommel 17 und dem Dosierglied 18 einerseits und dem zweiten Förderglied 12 andererseits besteht.

Nachdem die gewünschte Dosis eingestellt ist, beispielsweise die Maximaldosis, die im Ausführungsbeispiel 80 Einheiten beträgt (Figur 2), zieht der Benutzer die Kappe 4 ab, falls diese aus Sicherheitsgründen noch aufgesteckt war, sticht die Injektionsnadel an der gewünschten Einstechstelle durch die Haut und platziert die Nadelspitze so im subkutanen Gewebe.

Bei subkutan platzierter Injektionsnadel hält der Benutzer die Vorrichtung mit einer Hand im Bereich des Gehäuses 1, 2 und drückt mit dem Daumen in Förderrichtung V gegen das Betätigungselement 30. Durch den Druck wird in einer ersten Phase des Bestätigungshubs das zweite Kupplungsglied 22 gegen den Druck der Feder 20 relativ zu dem ersten Kupplungsglied 21 in Förderrichtung V aus dem Kupplungseingriff bewegt. Die axiale Länge der den Kupplungseingriff vermittelnden Eingriffselemente 24 und 25 ist so bemessen, dass der Kupplungseingriff gelöst ist, wenn das Betätigungselement 30 relativ zu dem Kupplungsglied 21 den Leerhub L_{H} zurückgelegt hat. Bei weiterem Druck auf das Betätigungselement 30 drückt dieses über das Kontaktelement 32 gegen das Kupplungsglied 21, so dass sich die Anzeigetrommel 17 in der zweiten Phase des Betätigungshubs im zweiten Gewindeeingriff 9, 19 in den Gehäuseabschnitt 2 schraubt. Das zweite Kupplungsglied 22 fährt dabei teleskopartig in der Ausgleichsstruktur 23 bis gegen einen axialen Anschlag, den die Ausgleichsstruktur 23 im Ausführungsbeispiel durch ihren Boden bildet. Sobald das Kupplungsglied 22 gegen die Ausgleichsstruktur 23 auf Anschlag gelangt, bewirkt der weitere Druck auf das Betätigungselement 30, dass nun in der letzten Phase des Betätigungshubs das zweite Förderglied 12 wegen der andrückenden Ausgleichsstruktur 23 mitgenommen wird und den Förderhub ausführt. Der Förderhub wird durch das Anschlagpaar 6, 14 begrenzt. Der Betätigungshub ist zwar funktional in Phasen gegliedert, wird vorteilhafter weise aber kontinuierlich ausgeführt.

Nach vollständiger Ausführung des Förderhubs wird der Druck von dem Betätigungselement 30 genommen, so dass die Feder 20 das Kupplungsglied 22 wieder in den Kupplungseingriff mit dem Kupplungsglied 21 drückt. Nachdem die Injektionsnadel aus dem Gewebe gezogen wurde, wird die Kappe 4 wieder aufgesteckt. Die Vorrichtung befindet sich nun wieder im Zustand der Figur 4, allerdings ohne die nur vor der erstmaligen Benutzung vorhandene Nadelschutzkappe. In diesem Zustand wird die Vorrichtung bis zur nächsten Injektion aufbewahrt.

### Bezugszeichen:

- 1: distaler Gehäuseabschnitt
- 2: proximaler Gehäuseabschnitt
- 3: Behältnis
- 4: Kappe
- 5: Rückhalteeinrichtung, Verdrehsicherung
- 6: Förderanschlag
- 7: Fenster
- 8: -
- 9: Gewinde
- 10: -
- 11: erstes Förderglied
- 12: zweites Förderglied
- 13: Gewinde
- 14: Förderarischlag
- 15: -
- 16: -
- 17: Anzeigetrommel
- 18: Dosierglied
- 19: Gewinde
- 20: Feder
- 21: erstes Kupplungsglied
- 22: zweites Kupplungsglied, Ausgleichsstruktur
- 23: Ausgleichsstruktur
- 24: Eingriffselemente, Rippen
- 25: Eingriffselemente, Nuten
- 26: Führungselemente, Rippen
- 27: Führungselemente, Nuten
- 28: -
- 29: -
- 30: Betätigungselement
- 31: Kontaktfläche
- 32: Kontaktelement

- V: Förderrichtung, Betätigungsrichtung
- L: Längsachse, Förderachse

## Patentansprüche

1. Verabreichungsvorrichtung mit Anzeigetrommel, umfassend:
a) ein Gehäuse (1, 2) mit einer Aufnahme für ein zu verabreichendes Produkt,
b) ein erstes Förderglied (11), das für die Förderung des Produkts relativ zu dem Gehäuse (1, 2) in eine Förderrichtung (V) entlang einer Förderachse (L) bewegbar ist,
c) ein zweites Förderglied (12), das in einem ersten Gewindeeingriff mit dem ersten Förderglied (11) relativ zu dem Gehäuse (1, 2) in und gegen die Förderrichtung (V), relativ zu dem Gehäuse (1, 2) und dem ersten Förderglied (11) rotatorisch und relativ zu dem ersten Förderglied (11) gegen die Förderrichtung (V) translatorisch bewegbar ist,
d) eine Dosiereinrichtung, mittels der eine Produktdosis einstellbar ist und die für die Anzeige der Produktdosis die Anzeigetrommel (17) umfasst, die in einem zweiten Gewindeeingriff relativ zu dem Gehäuse (1, 2) rotatorisch und in und gegen die Förderrichtung (V) translatorisch bewegbar ist,
e) und eine Kupplung (20-22), die das zweite Förderglied (12) und die Anzeigetrommel (17) in einem Kupplungseingriff verdrehgesichert miteinander verbindet, wobei die Kupplung (20-22) ein erstes Kupplungsglied (21), ein zweites Kupplungsglied (22) und eine das zweite Kupplungsglied (22) in den Kupplungseingriff mit dem ersten Kupplungsglied (21) spannende Feder umfasst, wobei der Kupplungseingriff durch Betätigung eines Betätigungselements (30) lösbar ist,
f) wobei die Gewindesteigung des zweiten Gewindeeingriffs grösser ist als die Gewindesteigung des ersten Gewindeeingriffs,
g) wobei das Betätigungselement (30) einen Verbindungsabschnitt aufweist, mit dem es mit dem zweiten Kupplungsglied (22) verbunden ist, wobei der Verbindungsabschnitt durch einen Boden des ersten Kupplungsglieds (21) in das Innere der Dosiereinrichtung ragt und in dem zweiten Kupplungsglied (22) verankert ist, so dass das zweite Kupplungsglied (22) und das Betätigungselement (30) in und gegen die Förderrichtung (V) relativ zueinander nicht beweglich sind, wobei das Betätigungselement (30) relativ zu dem ersten Kupplungsglied (21) um die Förderachse (L) drehbar ist, und wobei
h) eine Kontaktfläche (31) des Betätigungselements (30) um den Verbindungsabschnitt des Betätigungselements (30) zentral als in die distale Richtung aufragende, erhabene Fläche gebildet ist und
i) zur Bildung einer Kontaktgegenfläche in das erste Kupplungsglied (21) komplementär zur Kontaktfläche (31) ein Kontaktelement (32) eingesetzt ist.

2. Verabreichungsvorrichtung nach dem vorhergehenden Anspruch, **dadurch gekennzeichnet, dass** die Anzeigetrommel (17) mit einem der Kupplungsglieder (21, 22) verdrehgesichert verbunden ist oder das eine der Kupplungsglieder (21, 22) bildet.

3. Verabreichungsvorrichtung nach einem der zwei vorhergehenden Ansprüche, **dadurch gekennzeichnet, dass** die Feder (20) das zweite Kupplungsglied (22) in oder vorzugsweise gegen die Förderrichtung (V) in den Kupplungseingriff spannt.

4. Verabreichungsvorrichtung nach einem der zwei vorhergehenden Ansprüche, **dadurch gekennzeichnet, dass** das zweite Förderglied (12) und das zweite Kupplungsglied (22) längs einer gemeinsamen Achse (L) translatorisch bewegbar sind.

5. Verabreichungsvorrichtung nach einem der vorhergehenden Ansprüche, **dadurch gekennzeichnet, dass** der Kupplungseingriff formschlüssig ist.

6. Verabreichungsvorrichtung nach einem der vorhergehenden Ansprüche, **dadurch gekennzeichnet, dass** die Verabreichungsvorrichtung das Betätigungselement (30) für das zweite Förderglied (12) umfasst und wenigstens eines aus Anzeigetrommel (17) und zweitem Förderglied (12) relativ zu dem Betätigungselement (30) drehbar ist.

7. Verabreichungsvorrichtung nach dem vorhergehenden Anspruch, **dadurch gekennzeichnet, dass** das Betätigungselement (30) für die Betätigung des zweiten Förderglieds (12) relativ zu wenigstens einem aus Gehäuse (1, 2) und erstem Kupplungsglied (21) bewegbar ist und mit dem zweiten Kupplungsglied (22) so gekoppelt ist, dass es bei einer durch die Betätigung bewirkten Bewegung, die vorzugsweise in die Förderrichtung (V) stattfindet, das zweite Kupplungsglied (22) aus dem Kupplungseingriff bewegt.

8. Verabreichungsvorrichtung nach dem vorhergehenden Anspruch, **dadurch gekennzeichnet, dass** das Betätigungselement (30) nach dem Lösen des Kupplungseingriffs in einen Eingriff gelangt, in dem es bei weiterer Bewegung die Anzeigetrommel (17) mitnimmt.

9. Verabreichungsvorrichtung nach dem vorhergehenden Anspruch, **dadurch gekennzeichnet, dass** das Betätigungselement (30) in den Eingriff mit der Anzeigetrommel (17) erst gelangt, nachdem der Kupplungseingriff gelöst ist.

10. Verabreichungsvorrichtung nach einem der drei vorhergehenden Ansprüche, **dadurch gekennzeichnet, dass** das Betätigungselement (30) nach dem Lösen des Kupplungseingriffs über das zweite Kupplungsglied (22) in eine Wirkverbindung mit dem zweiten Förderglied (12) gelangt, in der es bei weiterer Bewegung das zweite Förderglied (12) mitnimmt.

11. Verabreichungsvorrichtung nach einem der vorhergehenden Ansprüche, umfassend eine Ausgleichseinrichtung (20, 22, 23), die in Förderrichtung (V) längenvariabel ist und bei bestehendem Kupplungseingriff das zweite Förderglied (12) mit der Anzeigetrommel (17) verdrehgesichert verbindet.

12. Verabreichungsvorrichtung nach dem vorhergehenden Anspruch, **dadurch gekennzeichnet, dass** eine Feder (20) vorgesehen ist, gegen deren Federkraft die Länge der Ausgleichseinrichtung (20, 22, 23) verringerbar ist.

13. Verabreichungsvorrichtung nach einem der zwei vorhergehenden Ansprüche, **dadurch gekennzeichnet, dass** die Ausgleichseinrichtung (20, 22, 23) ein Teleskop bildet.

14. Verabreichungsvorrichtung nach einem der drei vorhergehenden Ansprüche, **dadurch gekennzeichnet, dass** die Ausgleichseinrichtung (20, 22, 23) wenigstens zwei in Vortriebsrichtung (V) einander überlappende und relativ zueinander verdrehgesichert geführte Ausgleichsstrukturen (22, 23) umfasst und dass bei bestehendem Kupplungseingriff eine der Ausgleichsstrukturen (22, 23) mit der Anzeigetrommel (17) und die andere mit dem zweiten Förderglied (12) verdrehgesichert verbunden ist.

15. Verabreichungsvorrichtung nach dem vorhergehenden Anspruch, **dadurch gekennzeichnet, dass** eine Feder (20) vorgesehen ist, die in die Förderrichtung (V) auf eine der Ausgleichsstrukturen (22, 23) und gegen die Förderrichtung (V) auf die andere der Ausgleichsstrukturen (22, 23) wirkt.

16. Verabreichungsvorrichtung nach einem der zwei vorhergehenden Ansprüche, **dadurch gekennzeichnet, dass** das zweite Kupplungsglied (22) eine der Ausgleichsstrukturen (22, 23) bildet.

17. Verabreichungsvorrichtung nach einem der drei vorhergehenden Ansprüche, **dadurch gekennzeichnet, dass** die Feder (20) der Ausgleichseinrichtung (20, 22, 23) die Feder (20) der Kupplung (20-22) ist.

18. Verabreichungsvorrichtung nach einem der vorhergehenden Ansprüche, **dadurch gekennzeichnet, dass** eine Führung vorgesehen ist, mittels der das erste Förderglied (11) entlang einer in Vortriebsrichtung (V) weisenden Förderachse (L) bezüglich beider Drehrichtungen relativ zu dem Gehäuse (1, 2) nicht drehbar geführt wird oder führbar ist.

19. Verabreichungsvorrichtung nach einem der vorhergehenden Ansprüche, **dadurch gekennzeichnet, dass** eine Rückhalteeinrichtung (5) vorgesehen ist, um in einem Eingriff mit dem ersten Förderglied (11) eine Bewegung des ersten Förderglieds (11) gegen die Förderrichtung (V) zu verhindern.

20. Verabreichungsvorrichtung nach einem der vorhergehenden Ansprüche, **dadurch gekennzeichnet, dass** das erste Förderglied (11) eine Kolbenstange ist.

## Claims

1. An administering device comprising a display drum, including:
a) a housing (1, 2) comprising a receptacle for a product to be administered;
b) a first conveying member (11) which can be moved relative to the housing (1, 2) in a conveying direction (V) along a conveying axis (L) in order to convey the product;
c) a second conveying member (12) which in a first threaded engagement with the first conveying member (11) can be rotationally moved relative to the housing (1,2) and the first conveying member (11) in and in opposite relationship to the conveying direction (V) and translationally moved relative to the first conveying member (11) in opposite relationship to the conveying direction (V);
d) a dosing means, by means of which a product dosage can be set and which comprises the display drum (17) for displaying the product dosage, which display drum (17) in a second threaded engagement can be rotationally moved relative to the housing (1, 2) and translationally moved in and in opposite relationship to the conveying direction (V);
e) and a coupling (20-22) which connects the second conveying member (12) and the display drum (17) together in mutually rotationally secured relationship in a coupling engagement, wherein the coupling (20-22) comprises a first coupling member (21), a second coupling member (22) and a spring (20) which stresses the second coupling member (22) into the coupling engagement with the first coupling member (21), wherein the coupling engagement can be released by actuating an actuating element (30);
f) wherein the thread pitch of the second threaded engagement is greater than the thread pitch of the first threaded engagement,
g) wherein the actuating element (30) has a connecting portion with which it is connected to the second coupling member (22), wherein the connecting portion projects through a bottom of the first coupling member (21) into the interior of the dosing means and is anchored in the second coupling member (22) so that the second coupling member (22) and the actuating element (30) are not movable relative to each other in and in opposite relationship to the conveying direction (V), wherein the actuating element (30) is rotatable relative to the first coupling member (21) about the conveying axis (L), and wherein
h) a contact surface (31) of the actuating element (30) is formed around the connecting portion of the actuating element (30) centrally as a raised surface projecting in the distal direction, and
i) to form a counterpart contact surface a contact element (32) is inserted into the first coupling member (21) in complementary relationship with the contact surface (32)

2. An administering device according to the preceding claim **characterised in that** the display drum (17) is connected in mutually rotationally secured relationship to one of the coupling members (21, 22) or forms one of the coupling members (21, 22).

3. An administering device according to any one of the two preceding claims **characterised in that** the spring (20) stresses the second coupling member (22) into the coupling engagement in or preferably in opposite relationship to the conveying direction (V).

4. An administering device according to any one of the three preceding claims **characterised in that** the second conveying member (12) and the second coupling member (22) can be translationally moved along a common axis (L).

5. An administering device according to any one of the preceding claims **characterised in that** the coupling engagement is a positively locking engagement.

6. An administering device according to any one of the preceding claims **characterised in that** the administering device comprises an actuating element (30) for the second conveying member (12) and at least one of the display drum (17) and the second conveying member (12) can be rotated relative to the actuating element (30).

7. An administering device according to the preceding claim **characterised in that** the actuating element (30) can be moved relative to at least one of the housing (1, 2) and the first coupling member (21) in order to actuate the second conveying member (12), and is coupled to the second coupling member (22) such that it moves the second coupling member (22) out of the coupling engagement during a movement caused by actuation, which is preferably performed in the conveying direction (V).

8. An administering device according to the preceding claim **characterised in that** after the coupling engagement has been released the actuating element (30) passes into an engagement in which it entrains the display drum (17) upon further movement.

9. An administering device according to the preceding claim **characterised in that** the actuating element (30) does not pass into the engagement with the display drum (17) until after the coupling engagement has been released.

10. An administering device according to any one of the three preceding claims **characterised in that** after the coupling engagement has been released the actuating element (30) passes into an operative connection with the second conveying member (12) via the second coupling member (22), in which it entrains the second conveying member (12) upon further movement.

11. An administering device according to any one of the preceding claims comprising an equalisation means (20, 22, 23) which is variable in length in the conveying direction (V) and connects the second conveying member (12) to the display drum (17) in mutually rotationally secured relationship when the coupling engagement exists.

12. An administering device according to the preceding claim **characterised in that** a spring (20) is provided, wherein the length of the equalisation means (20, 22, 23) can be reduced against the spring force of said spring (20).

13. An administering device according to any one of the two preceding claims **characterised in that** the equalisation means (20, 22, 23) forms a telescope.

14. An administering device according to any one of the three preceding claims **characterised in that** the equalisation means (20, 22, 23) comprises at least two equalisation structures (22, 23) which overlap each other in the advancing direction (V) and are guided relative to each other in mutually rotationally secured relationship, and that one of the equalisation structures (22, 23) is connected to the display drum (17) in mutually rotationally secured relationship and the other is connected to the second conveying member (12) in mutually rotationally secured relationship when the coupling engagement exists.

15. An administering device according to the preceding claim **characterised in that** a spring (20) is provided which acts on one of the equalisation structures (22, 23) in the conveying direction (V) and acts on the other of the equalisation structures (22, 23) in opposite relationship to the conveying direction (V).

16. An administering device according to any one of the two preceding claims **characterised in that** the second coupling member (22) forms one of the equalisation structures (22, 23).

17. An administering device according to any one of the three preceding claims **characterised in that** the spring (20) of the equalisation means (20, 22, 23) is the spring (20) of the coupling (20-22).

18. An administering device according to any one of the preceding claims **characterised in that** a guide is provided, by means of which the first conveying member (11) is or can be non-rotationally guided relative to the housing (1, 2), with respect to both rotational directions, along a conveying axis (L) which points in the advancing direction (V).

19. An administering device according to any one of the preceding claims **characterised in that** a retaining means (5) is provided in order to prevent a movement of the first conveying member (11) in opposite relationship to the conveying direction (V) in an engagement with the first conveying member (11).

20. An administering device according to any one of the preceding claims **characterised in that** the first conveying member (11) is a piston rod.

## Revendications

1. Dispositif d'alimentation équipé d'un tambour d'affichage, comprenant :
a) un logement (1, 2) doté d'un réceptacle pour un produit à administrer
b) un premier élément d'alimentation (11), qui est mobile le long d'un axe d'alimentation (L) pour alimenter le produit par rapport au logement (1, 2) dans un sens d'alimentation (V),
c) un deuxième élément d'alimentation (12) qui est mobile dans un premier engagement à filetage avec le premier élément d'alimentation (11) par rapport au logement (1, 2) dans le sens de l'alimentation (V) et dans le sens opposé, en rotation par rapport au logement (1, 2) et au premier élément d'alimentation (11) et mobile en translation par rapport au premier élément d'alimentation (11) dans le sens opposé au sens d'alimentation (V),
d) un dispositif de dosage, au moyen duquel une dose de produit peut être réglée et qui comprend le tambour d'affichage (17) pour l'affichage de la dose de produit, lequel tambour est mobile en rotation dans un deuxième engagement à filetage par rapport au logement (1, 2), et mobile en translation dans le sens d'alimentation (V) et dans le sens opposé,
e) un accouplement (20 - 22), qui relie ensemble sans possibilité de rotation le deuxième élément d'alimentation (12) et le tambour d'affichage (17) dans un engagement par accouplement, l'accouplement (20 - 22) comprenant un premier élément d'accouplement (21), un deuxième élément d'accouplement (22) et un ressort serrant le deuxième élément d'accouplement (22) dans l'engagement par accouplement avec le premier élément d'accouplement (21), l'engagement par accouplement pouvant être détaché en actionnant un élément d'actionnement (30),
f) le pas du filetage du deuxième engagement à filetage étant plus grand que le pas du filetage du premier engagement à filetage,
g) l'élément d'actionnement (30) présentant une section de liaison, par laquelle il est relié au deuxième élément d'accouplement (22), la section de liaison dépassant dans l'espace intérieur du dispositif de dosage en passant par le fond du premier élément d'accouplement (21) et étant ancré dans le deuxième élément d'accouplement (22) de sorte que le deuxième élément d'accouplement (22) et l'élément d'actionnement (30) ne sont pas mobiles l'un par rapport à l'autre dans le sens d'alimentation (V) et dans le sens opposé, l'élément d'actionnement (30) pouvant tourner autour de l'axe d'alimentation (L) par rapport au premier élément d'accouplement (21), et
h) une surface de contact (31) de l'élément d'actionnement (30) étant formée de manière centrée autour de la section de liaison de l'élément d'actionnement (30) en tant qu'une surface surélevée dépassant dans la direction distale, et
i) un élément de contact (32) étant utilisé pour former une contre-surface de contact dans le premier élément d'accouplement (21) en complément de la surface de contact (31).

2. Dispositif d'administration selon la revendication précédente, **caractérisé en ce que** le tambour d'affichage (17) est relié sans possibilité de rotation à un des éléments d'accouplement (21, 22) ou forme un des éléments d'accouplement (21, 22).

3. Dispositif d'alimentation selon l'une quelconque des deux revendications précédentes, **caractérisé en ce que** le ressort (20) tend le deuxième élément d'accouplement (22) dans le sens d'alimentation (V), ou de préférence dans le sens opposé, dans l'engagement par accouplement.

4. Dispositif d'administration selon l'une quelconque des deux revendications précédentes, **caractérisé en ce que** le deuxième élément d'alimentation (12) et le deuxième élément d'accouplement (22) sont mobiles par translation le long d'un axe commun (L).

5. Dispositif d'administration selon l'une quelconque des revendications précédentes, **caractérisé en ce que** l'engagement par accouplement est à complémentarité de forme.

6. Dispositif d'administration selon l'une quelconque des revendications précédentes, **caractérisé en ce que** le dispositif d'administration comprend l'élément d'actionnement (30) pour le deuxième élément d'alimentation (12), et **en ce qu'**au moins un parmi le tambour d'affichage (17) et le deuxième élément d'alimentation (12) est pivotant par rapport à l'élément d'actionnement (30).

7. Dispositif d'administration selon la revendication précédente, **caractérisé en ce que** l'élément d'actionnement (30) pour actionner le deuxième élément d'alimentation (12) par rapport à au moins un parmi le logement (1, 2) et le premier élément d'accouplement (21) est mobile et couplé avec le deuxième élément d'accouplement (22) de telle sorte que lors d'un mouvement provoqué par l'actionnement, qui se produit de préférence dans le sens d'alimentation (V), le deuxième élément d'accouplement (22) est déplacé de l'engagement par accouplement.

8. Dispositif d'administration selon la revendication précédente, **caractérisé en ce que** l'élément d'actionnement (30) parvient à venir en prise une fois l'engagement par accouplement détaché, mise en prise dans laquelle il entraîne le tambour d'affichage (17) en cas de mouvement supplémentaire.

9. Dispositif d'administration selon la revendication précédente, **caractérisé en ce que** l'élément d'actionnement (30) vient en prise avec le tambour d'affichage (17) uniquement une fois que l'engagement par accouplement est détaché.

10. Dispositif d'administration selon l'une quelconque des trois revendications précédentes, **caractérisé en ce que** l'élément d'actionnement (30) parvient à une liaison fonctionnelle avec le deuxième élément d'alimentation (12) après avoir détaché l'engagement par accouplement sur le deuxième élément d'accouplement (22), liaison fonctionnelle dans laquelle il entraîne le deuxième élément d'alimentation (12) en cas de mouvement supplémentaire.

11. Dispositif d'administration selon l'une quelconque des revendications précédentes, comprenant un dispositif de compensation (20, 22, 23) qui peut varier en longueur dans le sens d'alimentation (V) et qui relie sans possibilité de rotation, en cas d'engagement par accouplement existant, le deuxième élément d'alimentation (12) au tambour d'affichage (17).

12. Dispositif d'alimentation selon la revendication précédente, **caractérisé en ce qu'**un ressort (20) est prévu, contre la force duquel la longueur du dispositif de compensation (20, 22, 23) peut être réduite.

13. Dispositif d'administration selon l'une quelconque des deux revendications précédentes, **caractérisé en ce que** le dispositif de compensation (20, 22, 23) forme un télescope.

14. Dispositif d'alimentation selon l'une quelconque des trois revendications précédentes, **caractérisé en ce que** le dispositif de compensation (20, 22, 23) comprend au moins deux structures de compensation (22, 23) se chevauchant l'une dans l'autre dans la direction de propulsion (V) et guidées l'une par rapport à l'autre sans possibilité de rotation, et **caractérisé en ce que**, en cas d'engagement par accouplement existant, une des structures de compensation (22, 23) est reliée au tambour d'affichage (17) et l'autre est reliée sans possibilité de rotation au deuxième élément d'alimentation (12).

15. Dispositif d'administration selon la revendication précédente, **caractérisé en ce qu'**un ressort (20) est prévu qui, dans le sens d'alimentation (V) agit sur une des structures de compensation (22, 23) et, dans le sens opposé au sens d'alimentation (V), agit sur l'autre des structures de compensation (22, 23).

16. Dispositif d'administration selon l'une quelconque des deux revendications précédentes, **caractérisé en ce que** le deuxième élément d'accouplement (22) constitue une des structures de compensation (22, 23).

17. Dispositif d'administration selon l'une quelconque des trois revendications précédentes, **caractérisé en ce que** le ressort (20) du dispositif de compensation (20, 22, 23) est le ressort (20) de l'accouplement (20 - 22).

18. Dispositif d'administration selon l'une quelconque des revendications précédentes, **caractérisé en ce qu'**un système de guidage est prévu, au moyen duquel le premier élément d'alimentation (11) est guidé ou peut être guidé de façon non pivotante le long d'un axe d'alimentation (L) dirigé dans le sens de propulsion (V) concernant les deux sens de rotation par rapport au logement (1, 2).

19. Dispositif d'administration selon l'une quelconque des revendications précédentes, **caractérisé en ce qu'**un dispositif de retenue (5) est prévu pour empêcher, dans un engagement avec le premier élément d'alimentation (11), tout mouvement du premier élément d'alimentation (11) dans le sens opposé au sens d'alimentation (V).

20. Dispositif d'administration selon l'une quelconque des revendications précédentes, **caractérisé en ce que** le premier élément d'alimentation (11) est une tige de piston.
